# EUROPÄISCHE PATENTSCHRIFT

(11) **EP 1 839 027 B1**
(45) Veröffentlichungstag und Bekanntmachung des Hinweises auf die Patenterteilung: **19.10.2016**
(21) Anmeldenummer: 05803117.0
(22) Anmeldetag: 18.11.2005
(51) Int. Cl.: G01N 1/28, B02C 19/00, B01F 13/10, B02C 18/16

(54) **EINWEGMISCHER, -HOMOGENISATOR, -EXTRAHIERER, -FRAKTIONIERER ODER -AUFSCHLÄMMER**
ONE-WAY MIXER, HOMOGENISER, EXTRACTOR, FRACTIONER OR SLURRY PRODUCER
MELANGEUR, HOMOGENEISATEUR, EXTRACTEUR, APPAREIL DE FRACTIONNEMENT OU APPAREIL DE MISE EN SUSPENSION A USAGE UNIQUE

(30) Priorität: 21.01.2005 CH 922005; 07.11.2005 CH 17822005
(43) Veröffentlichungstag der Anmeldung: 03.10.2007
(73) Patentinhaber: Medic Tools AG, 6300 Zug (CH)
(72) Erfinder: BUCHER, Franz, Gregor, CH-6304 Zug (CH)
(74) Vertreter: Kisters, Michael Marcus
(86) Internationale Anmeldenummer: PCT/CH2005/000685
(87) Internationale Veröffentlichungsnummer: WO 2006/076820

(56) Entgegenhaltungen:
- EP-A- 0 590 219
- WO-A-2004/035191
- US-A- 3 941 317

## Beschreibung

Gegenstand der Erfindung ist ein Einwegmischer, -homogenisator, -extrahierer, -fraktionierer oder -aufschlämmer gemäss Oberbegriff des Patentanspruchs 1.

Im Laborbetrieb sind verschiedene Vorrichtungen zum Mischen, Homogenisieren, Extrahieren, Fraktionieren oder Aufschlämmen von meist in Kleinmengen zu bearbeitenden Stoffen, insbesondere infektiösen, übelriechenden, chemisch aggressiven oder steril zu haltenden Stoffen, bekannt.

Aus der EP-B1 1361917 ist eine solche Vorrichtung bekannt, bei der das Labor-Testgerät und das Rührelement eine Einheit bilden und es ermöglichen, dass während des Bearbeitungsvorgangs des Stoffes dieser nicht austreten und die Umgebung kontaminieren kann. Das bekannte Gerät ermöglicht folglich, die Bearbeitung des Stoffes in einem geschlossenen Behälter durchzuführen und so durch den hermetischen Verschluss eine Infektion, unkontrollierte Spritzer oder Ausschütten durch unbeabsichtigtes Umkippen des Labor-Testgerätes zu vermeiden.

Ein gattungsgemässer Fraktionierer ist aus der US-A-3941317 bekannt. Ausgehend von diesem Stand der Technik ist es Aufgabe der vorliegenden Erfindung, einen Einwegmischer, -homogenisator, -extrahierer, -fraktionierer oder -aufschlämmer zu schaffen, welcher mit einfachen und kostengünstigen Mitteln der Anpresskraftausübung eine Automatisierung des Fraktionierprozesses ermöglicht.

Diese Aufgabe wird erfindungsgemäss durch einen Einwegmischer, -homogenisator, -extrahierer, -fraktionierer oder -aufschlämmer gemäss den Merkmalen des Patentanspruchs 1 gelöst.

Vorteilhafte Ausgestaltungen der Vorrichtung sind in den abhängigen Ansprüchen umschrieben.

Durch den erfindungsgemässen Aufbau der Vorrichtung gelingt es, nach dem Einbringen des zu bearbeitenden Stoffes in den Behälter, z.B. bereits am Ort, wo der Stoff anfällt, dessen Verarbeitung vollständig hermetisch und von der Aussenwelt abgeschlossen innerhalb der Vorrichtung durchzuführen und anschliessend das Produkt aus der Verarbeitung ohne öffnen der Vorrichtung, d.h. ohne Abnehmen des Deckels vom Labor-Testgerät, dem Gerät zu entnehmen. Die Vorrichtung kann für unterschiedliche Arbeiten eingesetzt werden. Je nach Lochgrösse und Form des als Raffelschneider fungierenden Siebes können entsprechend gewünschte Fraktionen erreicht werden. Der zu bearbeitende Stoff ist während der Bearbeitung durch eine dichte Membran von der Umgebung getrennt und die Membran hält diese Trennung aufrecht, auch wenn nach der Entnahme des Stoffes das Entnahmeröhrchen der Pipette wieder herausgezogen wird. Mit dem elastisch federnden Niederpresser kann der zu bearbeitende Stoff sorgfältig gegen das Sieb gepresst werden und wird vom rotierenden Niederpresser sukzessive durch die Bohrungen im Sieb hindurchgeführt. Teile des Stoffes, die grösser sind als die Lochquerschnitte des Siebes werden über dem Sieb zurückgehalten, so dass unterhalb des Siebes nur die gewünschte weiter zu bearbeitende Stofffraktion sich ansammelt und von dort direkt entnommen werden kann. Die Vorrichtung besteht vorzugsweise vollständig aus Kunststoff und kann als solche zusammen mit den noch zurückgebliebenen, nicht benötigten oder nicht brauchbaren Stoffteilen entsorgt werden. Der Dreh-Antrieb für den Niederpresser, oder in einer anderen Ausgestaltung der Erfindung des Behälters, wird bei der Bearbeitung des Stoffes nicht verschmutzt und muss daher nicht gereinigt werden.

Der erfindungsgemässe Einwegmischer kann beispielsweise direkt in einem Schlachthof nach der Entnahme von Gewebeproben von Tieren mit diesen gefüllt und so die Gewebeprobe unter sterilen Bedingungen ins Labor gebracht werden. Die für die weitere Bearbeitung der Gewebeprobe notwendige Pufferlösung kann durch die Deckelplatte und die dort angebrachte Membran im Labor nachträglich noch zugegeben werden.

Der Einwegmischer ermöglicht es auch, da der darin bearbeitete Inhalt stets hermetisch abgeschlossen ist, die Fraktion nach dem Fraktionieren im Mischer zu belassen, dort Inkubieren (Wachsen) lassen und erst später zur Analyse zu entnehmen. Damit wird auch verhindert, dass das zu untersuchende Gewebe zwischen dem Fraktionieren und der Untersuchung von einem Gefäss zum andern gefördert werden muss.

Anhand eines illustrierten Ausführungsbeispiels wird die Erfindung näher erläutert. Es zeigen
- Figur 1: einen Axialschnitt durch den Behälter der Vorrichtung,
- Figur 2: eine Aufsicht auf den Behälter in Figur 1,
- Figur 3: eine Seitenansicht des Siebes,
- Figur 4: eine Aufsicht auf das Sieb in Figur 3,
- Figur 5: einen Axialschnitt durch den Behälter mit eingesetztem Sieb,
- Figur 6: einen Axialschnitt durch den Niederpresser,
- Figur 7: eine Untersicht des Niederpressers,
- Figur 8: einen Axialschnitt durch den Behälter mit eingesetztem Sieb und Niederpresser,
- Figur 9: einen Antrieb für den Behälter,
- Figur 10: einen Antrieb für den Niederpresser,
- Figur 11: eine Explosionsdarstellung der einzelnen Elemente des Einwegmischers mit Behälter oben,
- Figur 12: eine Explosionsdarstellung der einzelnen Elemente des Einwegmischers mit Behälter unten,
- Figur 13: der zusammengestellte Einwegmischer mit Behälter oben und
- Figur 14: der zusammengestellte Einwegmischer mit Behälter unten.

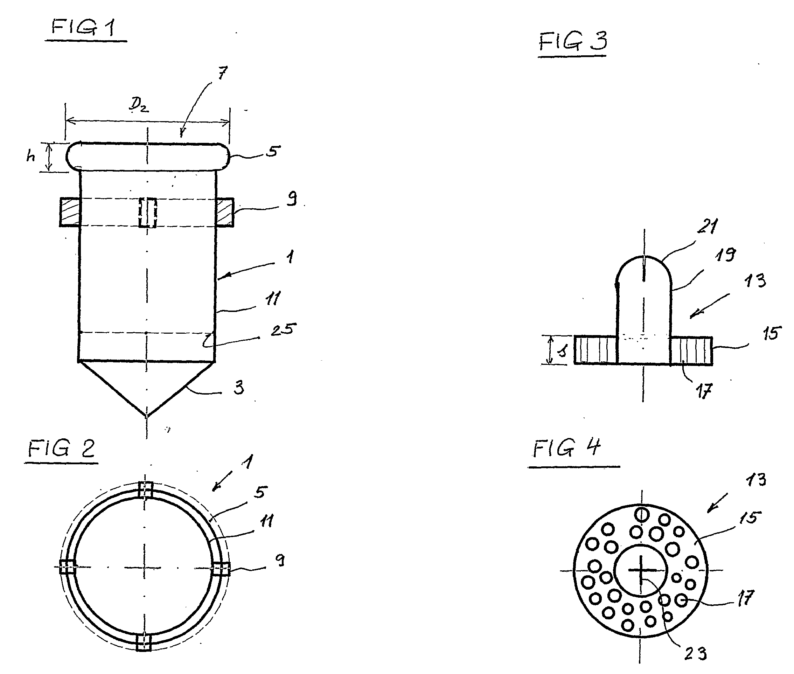

In Figur 1 ist ein zylindrisches Labor-Testgerät, im folgenden kurz Behälter 1 genannt, mit konisch verlaufendem Boden 3 und einem wulstförmigen Rand 5 im Bereich der öffnung 7 dargestellt. Beabstandet zum Rand 5 sind verteilt am Mantel 11 Haltenocken 9 angeordnet. Es können selbstverständlich auch mehr oder weniger als vier Nocken 9 vorhanden sein. Der zylindrische Mantel 11 und der Boden 3 des Behälters 1 sind der besseren Übersichtlichkeit halber nur mit einer einzigen Linie dargestellt. Die Wandstärke ist von der Grösse des Behälters und der Qualität des Kunststoffs abhängig. Der Behälter 1 ist vorzugsweise aus transparentem Kunststoff hergestellt, so dass der Zustand des zu verarbeitenden Stoffes sichtbar ist. Anstelle eines konisch verlaufenden Bodens 3 am Behälter 1 kann auch ein halbkugelförmiger Boden vorgesehen sein.

Die Figuren 3 und 4 zeigen ein Sieb 13, das als Teil eines Raffelschneiders dient und eine kreisringförmige Platte 15 mit im wesentlichen axial verlaufenden Löchern 17 umfasst. Die Löcher 17 können alle den selben Durchmesser aufweisen oder es können Löcher 17 mit unterschiedlichen Durchmessern mit unregelmässiger Verteilung über die Kreisringoberfläche eingesetzt werden (vgl. insbesondere Figur 4).

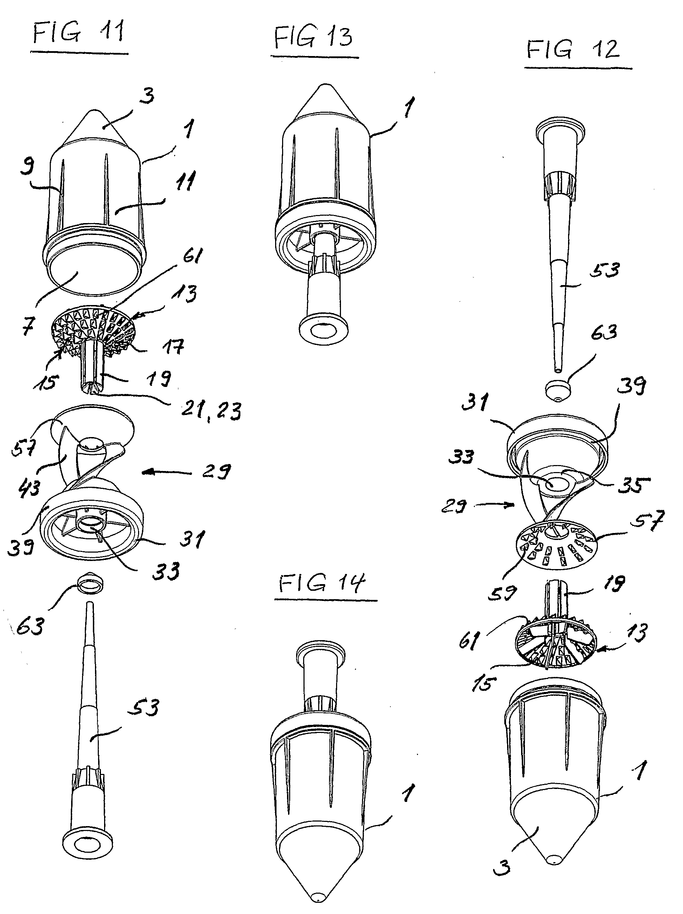

Im Zentrum der kreisringförmigen Platte 15 mit den Löchern 17 ist ein nach oben ragender Zylinder 19 angeordnet, welcher oben durch eine halbkugelförmige Haube 21 verschlossen ist. Die Haube 21 kann konkav (Fig. 11) oder konvex (Fig. 3) ausgebildet sein und besteht aus einem von einer Pipette durchstechbaren Material oder sie ist, wie in den Figuren dargestellt, durch kreuzförmig liegende Einschnitte 23 derart ausgebildet, dass eine Pipette 53 ohne grosse Kraft durch die Haube 21 hindurchgestossen werden kann. Die Einschnitte 23 sind vorzugsweise derart fein ausgebildet, dass kein zu verarbeitender Stoff, z.B. Gewebe, Gewebeflüssigkeit und dgl., in grösseren Mengen von oben nach unten durchtreten kann.

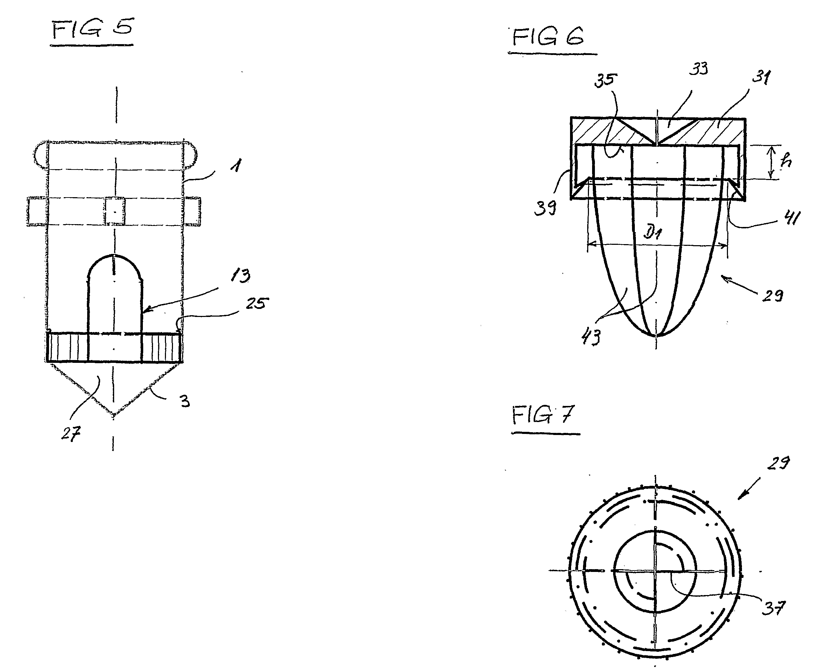

Die Platte 15 kann, wie in den Figuren 3 und 5 dargestellt, im Verhältnis zu deren Durchmesser eine grosse Höhe s aufweisen; sie kann aber auch im Gegensatz dazu (Fig. 11 und 12) wesentlich dünner ausgebildet sein. Das Sieb 13 mit dem zylindrischen Dom ist vorzugsweise aus Kunststoff hergestellt. Zum Fraktionieren von Knorpelmaterial kann das Sieb 13 auch aus Metall bestehen.

Die Figur 5 zeigt den Behälter 1 mit über dessen Boden 3 eingesetztem Sieb 13. Der Durchmesser des Siebes 13 ist derart bemessen, dass es sich in den zylindrischen Behälter 1 einschieben lässt und, wenn es unten angelangt ist, reibschlüssig gehalten wird. Zusätzlich können, um die Haltekraft des Siebes in der vorgegebenen Stellung sicherzustellen, an der Innenseite des Mantels 11 des Behälters 1 Nocken 25 über den Umfang verteilt angeordnet sein. Anstelle von Nocken 25 könnte auch eine umlaufende Rippe vorgesehen sein, welche das Sieb 13 an der ihm zugeordneten Stelle sicherstellt. Aus Figur 5 ist weiter sichtbar, dass unterhalb des Siebes 13 ein pyramidenförmiger, oder bei halbkugelförmigem Boden 3 ein entsprechend halbkugeliger Sammelraum 27 entsteht.

Die Figuren 6,7 und 11,12 zeigen einen Niederpresser 29, der gleichzeitig die Funktion eines Deckels für den Behälter 1 einnimmt. An einer kreisförmigen Deckelplatte 31 ist in deren Zentrum beispielsweise eine kegelförmige Ausnehmung 33 ausgebildet, deren Spitze annähernd bis zur Unterseite 35 der Deckelplatte 31 reicht. Radial verlaufende Einschnitte 37 ermöglichen es, die Deckelplatte 31 mit einer Pipette mit wenig Widerstand zu durchstossen. Alternativ zu den Einschnitten 37 könnte im Zentrum der Deckelplatte 31 ein Bereich der Deckplatte 31 membranartig dünn ausgeführt sein.

An der Peripherie der Deckelplatte 31 ist ein hohlzylindrischer Mantelabschnitt 39 angeformt, an dessen freier Kante ein nach innen ragender Bördel 41 angeordnet ist. Der Durchmesser D1 des Bördels 41 an dessen freier, konisch sich verengenden Kante ist kleiner als der Durchmesser D2 des umlaufenden Randes 5 am Behälter 1. Der Abstand der freien Kante des Bördels 41 von der Unterseite 35 der Deckelplatte 31 entspricht etwa der Höhe h der axialen Ausdehnung des Randes 5 an seiner Wurzel.

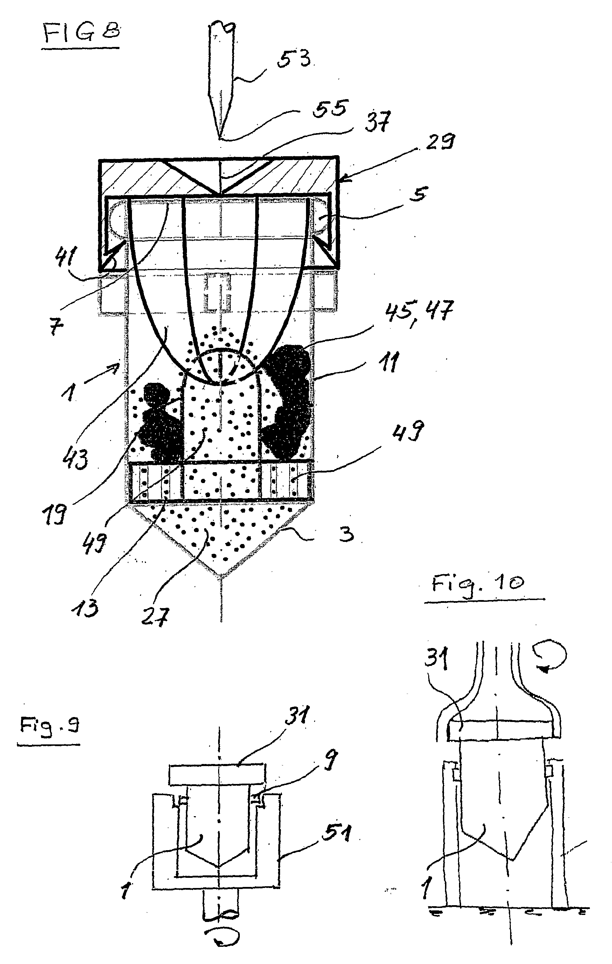

An der Unterseite 35 der Deckelplatte 31 sind weiter elastische, von der Deckelplatte 31 wegragende Elemente 43 angeordnet, welche den Mantelabschnitt 39 wesentlich überragen. Die elastischen Elemente 43 können auch streifenförmige Lappen oder schraubenlinienförmig verlaufende Stäbe sein. Die Funktion der elastischen Elemente 43 wird nachfolgend anhand der Figur 8, welche die zusammengesetzte Vorrichtung als Gesamtes darstellt, erläutert.

Die öffnung 7 des Behälters 1 ist durch die Deckelplatte 31 des Niederpressers 29 verschlossen. Beim Aufsetzen des Niederpressers 29 auf die öffnung 7 des Behälters 1 gleitet der Bördel 41 über den Rand 5 hinweg. Die Kanten des Bördels 41 untergreifen nun den Rand 5 und halten den Niederpresser 29 dichtend verbunden am Behälter 1. Die elastischen Elemente 43 ragen in den Behälterinnenraum hinein und pressen die vor dem Aufsetzen des Niederpressers 29 in den Behälter 1 eingefüllten zu verarbeitenden Stoffe am Zylinder 19 vorbei in den Ringraum zwischen dem Mantel des Zylinders 19 und dem Mantel 11 des Behälters an die Oberfläche des Siebes 13.

Die schwarzen Flächen 47 in Figur 8 bedeuten unverarbeitete Stoffe 45, die schwarzen Punkte 49 sind die bereits verarbeiteten Stoffe 45.

Die Figuren 11 bis 14 zeigen Ansichten des Einwegmischers in einer weiteren Ausgestaltung der Erfindung. Insbesondere der Niederpresser 29 ist in dieser Ausführungsform mit einer Reibplatte 57 versehen, welche eine konkave Reibfläche mit Reibzähnen 59 umfasst. Die aus dem ersten Ausführungsbeispiel mit Bezugszeichen 43 bezeichneten Elemente 43 sind schraubenlinienförmig angeordnet und tragen die Reibplatte 57 axial elastisch federnd an der Deckelplatte 31. Die Reibzähne 59 gelangen, wenn der Einwegmischer zusammengesetzt ist, in federelastische Anlage mit den an der Platte 15 des Siebes 13 ausgebildeten Zähnen oder Erhebungen 61. Die Erhebungen 61 und die Löcher 17 in der Platte 15 können in regelmässigen oder unregelmässigen Abständen zueinander angebracht sein. Weiter ist im zweiten Ausführungsbeispiel insbesondere in Figur 11 ein Verschlussdeckel 63 dargestellt, welcher die Ausnehmung 33 im Niederpresser 29 verschliesst. Der Verschlussdeckel 63 weist eine von der Pipette 53 durchstechbare Deckelfläche auf.

Im folgenden wird die Funktionsweise der Vorrichtung näher erläutert.
Die Bedienungsperson füllt durch die öffnung 7 zu bearbeitende Stoffe 45 in den Behälter 1. Zu bearbeitende Stoffe 45 können sein: menschliche oder tierische Gewebe, welche infektiös sind, übel riechen. Es können aber auch andere Feststoffe sein, die beispielsweise für eine Analyse in kleinere Fraktionen aufgelöst oder mit einer Flüssigkeit, die ebenfalls in den Behälter 1 zugegeben werden kann, intensiv vermischt werden müssen.

Nach dem Einfüllen des oder der Stoffe 45 in den Behälter 1 wird der Niederpresser 29 auf die öffnung 7 aufgeschoben und der Bördel 41 rastet unter dem Rand 5 ein. Beim Aufsetzen des Niederpressers 29 wird der Inhalt von den elastischen Elementen 43 über die Haube 21 des Zylinders 19 in den Ringraum gedrückt und kommt auf der Oberfläche des Siebes 13 in Anlage. Beim zweiten Ausführungsbeispiel wird der Inhalt von der Reibplatte 57 an das Sieb 13 angepresst. Nun wird die ganze Vorrichtung auf einen geeignet ausgebildeten Antrieb 51, der becherförmig ausgebildet sein kann und an dem die Nocken 9 einrasten können, aufgesetzt. Entweder wird nun die Deckelplatte 31 von Hand festgehalten und der Antrieb 51 in Gang gesetzt, oder es wird von oben ein Halteelement (nicht dargestellt) auf die Deckelplatte 31 abgesenkt, um diese festzuhalten. Durch den Antrieb 51 wird nun der Behälter 1 gedreht und der Stoff 47 durch die stillstehenden elastischen Elemente 43 durch die Löcher 17 in den Sammelraum 27 gepresst oder es wird der Behälter 1 gedreht und der Niederpresser 29 festgehalten. Es können folglich nur solche Fraktionen des Stoffes in den Sammelraum gelangen, die entweder kleiner sind als der Durchmesser der Löcher 17 oder solche, die durch die scharfen Kanten der Löcher 17 oder durch die Reibzähne 59 von grösseren Fraktionen abgetrennt worden sind. Wenn nun die kleinen Fraktionen 49 mit der meist zugegebenen Flüssigkeit in den Sammelraum 27 gelangen, wird dieser sukzessive aufgefüllt und das Gemisch steigt im Zylinder 19 wegen des Druckes der elastischen Elemente 49 auf die im Behälter 1 befindlichen Stoffe 45 nach oben. Je nach Ausbildung der Schlitze 37 in der Haube 21 können ein Teil der Fraktionen 49 oder mindestens die Flüssigkeit wieder oben austreten und werden ein zweites Mal durch das Sieb 13 hindurchgepresst. Sehnen und andere nicht fragmentierbare Teile verbleiben im oberen Teil des Behälters. Im unteren Teil, dem Sammelraum 27, sind folglich nur die erwarteten bearbeiteten Produkte. Diese können nun mit einer Pipette 53 ohne öffnen des Behälters 1 letzterem entnommen werden. Die Spitze 55 der Pipette 53 wird erst durch die Einschnitte 37 in der Deckelplatte 19 und dann durch die Einschnitte 23 in der Haube 21 des Zylinders 19 bis in den Sammelraum 27 vorgeschoben. Nun kann die Entnahme des aufbereiteten Stoffes ohne öffnen des Behälters 1 erfolgen. Die restlichen, nicht benötigten Teile werden zusammen mit der Vorrichtung entsorgt. Sie gelangen folglich, nachdem sie einmal in den Behälter 1 eingebracht worden sind, nie wieder in die Umgebung.

## Patentansprüche

1. Einwegmischer, -homogenisator, -extrahierer, -fraktionierer oder -aufschlämmer von Stoffen, insbesondere infektiösen, übelriechenden, chemisch aggressiven oder steril zu haltenden Stoffen, umfassend einen Behälter (1) und ein Bearbeitungsmittel (13),
im Behälter (1) ein Sieb (13) als Raffelschneider in einem Abstand zum Boden (3) des Behälters (1) unter Bildung eines Sammelraums (27) eingesetzt ist, **dadurch gekennzeichnet, dass**
durch die Behälteröffnung (7) ein drehbar gelagerter, federnd ausgebildeter Niederpresser (29) zum Niederpressen des am Sieb (13) zu verarbeitenden Stoffs (45) in den Behälter (1) hineinragt.

2. Einwegmischer nach Anspruch 1, **dadurch gekennzeichnet, dass** der Niederpresser (29) drehbar auf der öffnung des Behälters (1) gehalten wird.

3. Einwegmischer nach Anspruch 2, **dadurch gekennzeichnet, dass** der Niederpresser (29) eine Deckelplatte (31) umfasst, an der ein Mantelabschnitt (39) angeformt ist, welcher den Rand (5) des Behälters (1) umgreift und den Niederpresser (29) drehbar mit dem Behälter (1) verbindet.

4. Einwegmischer nach Anspruch 3, **dadurch gekennzeichnet, dass** das Sieb (13) mit einem in dessen Zentrum angeordneten, oben durch eine durchsteckbare Haube (21) verschlossenen Zylinder (19) eingesetzt ist.

5. Einwegmischer nach einem der Ansprüche 1 bis 4,
**dadurch gekennzeichnet, dass** am Niederpresser (29) elastische Elemente (43) angeordnet sind, welche sich am Sieb (13) anlegen und darauf liegende Stoffe (45) durch das Sieb (13) pressen.

6. Einwegmischer nach einem der Ansprüche 1 bis 4,
**dadurch gekennzeichnet, dass** am Niederpresser (29) elastische Elemente (43) angebracht sind, an deren Ende eine Reibplatte (57) befestigt ist, welche sich am Sieb (13) anlegt und zwischen Sieb (13) und Reibplatte (57) eingeschlossene Stoffe (45) zerreibt und durch das Sieb (13) presst.

7. Einwegmischer nach Anspruch 5 oder 6, **dadurch gekennzeichnet, dass** die elastischen Elemente (43) aus streifenförmigen Lappen oder schraubenlinienförmig verlaufenden Stäben bestehen.

8. Einwegmischer nach einem der Ansprüche 1 bis 7,
**dadurch gekennzeichnet, dass** das Sieb (13) Löcher (17) mit gleichen oder unterschiedlichen Durchmessern aufweist.

9. Einwegmischer nach einem der Ansprüche 1 bis 8,
**dadurch gekennzeichnet, dass** sowohl die Deckelplatte (31) des Niederpressers (29) als auch das Sieb (13) einen axial durchstechbaren Bereich (23,37) aufweisen.

10. Einwegmischer nach Anspruch 4, **dadurch gekennzeichnet, dass** das Sieb (13) von Nocken (25) gegen Verdrehung gesichert ist.

## Claims

1. A single-use mixer, homogeniser, extractor, fractionater, or slurry producer of substances, in particular infectious, malodorous or chemically corrosive substances, or substances that are to be kept sterile, comprising a vessel (1) and a processing means (13),
a sieve (13) being inserted in the vessel (1) as a cutter and being located at a distance from the floor (3) of the vessel (1), forming a collection chamber (27),
**characterised in that**, through the vessel opening (7), a rotatably supported, resilient compression element (29) protrudes into the vessel (1) to compress the substance (45) that is to be processed against the sieve (13).

2. The single-use mixer according to claim 1,
**characterised in that** the compression element (29) is held rotatably at the opening of the vessel (1).

3. The single-use mixer according to claim 2,
**characterised in that** the compression element (29) comprises a lid plate (31) on which a casing portion (39) is integrally formed, which engages around the rim (5) of the vessel (1) and connects the compression element (29) to the vessel (1) rotatably.

4. The single-use mixer according to claim 3,
**characterised in that** the sieve (13) is inserted via a cylinder (19) which is arranged in the centre of the sieve and which is closed at the top by a cap (21) that can be pierced.

5. The single-use mixer according to any one of claims 1 to 4, **characterised in that** elastic elements (43) are arranged at the compression element (29) which contact the sieve (13) and press substances (45) located thereupon through said sieve (13).

6. The single-use mixer according to any one of claims 1 to 4, **characterised in that** elastic elements (43) are mounted on the compression element (29), with a milling plate (57) being fastened at ends of said elastic elements, which milling plate contacts the sieve (13) and grinds substances (45) trapped between the sieve (13) and the milling plate (57) and presses them through the sieve (13).

7. The single-use mixer according to claim 5 or 6, **characterised in that** the elastic elements (43) consist of strip-shaped tabs or helically extending rods.

8. The single-use mixer according to any one of claims 1 to 7, **characterised in that** the sieve (13) is provided with holes (17) having equal or different diameters.

9. The single-use mixer according to any one of claims 1 to 8, **characterised in that** both the lid plate (31) of the compression element (29) as well as the sieve (13) are provided with an area (23, 27) that can be axially pierced.

10. The single-use mixer according to claim 4, **characterised in that** the sieve (13) is protected against twisting by cams (25).

## Revendications

1. Mélangeur à usage unique, homogénéisateur, extracteur, appareil de fractionnement ou de mise en suspension de substances, en particulier de substances infectieuses, malodorantes, chimiquement agressives ou à garder stériles, comprenant un contenant(1) et un moyen de traitement (13),
un tamis (13) en tant que râpe étant mis en place dans le contenant (1) à une distance du fond (3) du contenant (1) en formant une chambre collectrice (27),
**caractérisé en ce qu'**un dispositif de compression (29), réalisé de manière élastique, monté de manière rotative à travers l'ouverture de contenant (7) pour comprimer la substance (45) à travailler sur le tamis (13) dépasse dans le contenant (1).

2. Mélangeur à usage unique selon la revendication 1,
**caractérisé en ce que** le dispositif de compression (29) est maintenu de manière rotative sur l'ouverture du contenant (1).

3. Mélangeur à usage unique selon la revendication 2,
**caractérisé en ce que** le dispositif de compression (29) comprend une plaque de couverture (31) sur laquelle une partie d'enveloppe (39) est moulée, laquelle entoure le bord (5) du contenant (1) et relie le dispositif de compression (29) de manière rotative au contenant (1).

4. Mélangeur à usage unique selon la revendication 3,
**caractérisé en ce que** le tamis (13) est mis en place avec un cylindre fermé (19) disposé dans son centre, fermé en-haut par une calotte (21) pouvant être traversée.

5. Mélangeur à usage unique selon l'une des revendications 1 à 4, **caractérisé en ce que** des éléments élastiques (43) sont disposés sur le dispositif de compression (29), lesquels arrivent à reposer contre le tamis (13) et pressent à travers le tamis (13) des substances (45) reposant sur celui-ci.

6. Mélangeur à usage unique selon l'une des revendications 1 à 4, **caractérisé en ce que** des éléments élastiques (43) sont aménagés sur le dispositif de compression (29), à l'extrémité desquels une plaque de friction (57) est fixée, laquelle arrive à reposer contre le tamis (13) et triture des substances (45) enfermées entre le tamis (13) et la plaque de friction (57) et les presse à travers le tamis (13).

7. Mélangeur à usage unique selon la revendication 5 ou 6, **caractérisé en ce que** les éléments élastiques (43) se composent de languettes en forme de bandes ou de tiges s'étendant en forme d'hélice.

8. Mélangeur à usage unique selon l'une des revendications 1 à 7, **caractérisé en ce que** le tamis (13) présente des trous (17) avec des diamètres égaux ou différents.

9. Mélangeur à usage unique selon l'une des revendications 1 à 8, **caractérisé en ce que** la plaque de couverture (31) du dispositif de compression (29) tout comme également le tamis (13) présentent une zone pouvant être perforée axialement (23, 37).

10. Mélangeur à usage unique selon la revendication 4, **caractérisé en ce que** le tamis (13) est protégé contre la torsion par des mentonnets (25).
